# EUROPEAN PATENT SPECIFICATION

(11) **EP 3 417 052 B1**
(45) Date of publication and mention of the grant of the patent: **15.02.2023**
(21) Application number: 17753829.5
(22) Date of filing: 16.02.2017
(51) Int. Cl.: C12N 5/0781, C12N 5/077

(54) **METHODS FOR EXPANDING AND DIFFERENTIATING B CELLS FOR PRODUCING ANTIBODY**
VERFAHREN ZUR EXPANSION UND DIFFERENZIERUNG VON B-ZELLEN ZUR ANTIKÖRPERHERSTELLUNG
PROCÉDÉS D'EXPANSION ET DE DIFFÉRENCIATION DE CELLULES B DESTINÉS À LA PRODUCTION D'ANTICORPS

(30) Priority: 16.02.2016 US 201662295728 P
(43) Date of publication of application: 26.12.2018
(73) Proprietor: Duke University, Durham, North Carolina 27705 (US)
(72) Inventor: SUGA, Hiraku, Durham NC 27710 (US); CANDANDO, Kathleen, M., Durham NC 27710 (US); KOUNTIKOV, Evgueni, Durham NC 27710 (US); KAMATA, Masahiro, Durham NC 27710 (US); TEDDER, Thomas, F., Durham NC 27705-1937 (US); YOSHIZAKI, Ayumi, Durham NC 27710 (US); MIYAGAKI, Tomomitsu, Durham NC 27710 (US)
(74) Representative: Mewburn Ellis LLP
(86) International application number: PCT/US2017/018155
(87) International publication number: WO 2017/143052

(56) References cited:
- EP-A1- 2 495 312
- WO-A2-2005/037218
- US-A1- 2014 065 118
- MASAHIRO KITABATAKE ET AL: "JNK Regulatory Molecule G5PR Induces IgG Autoantibody-Producing Plasmablasts from Peritoneal B1a Cells", THE JOURNAL OF IMMUNOLOGY, vol. 194, no. 4, 19 January 2015 (2015-01-19), pages 1480-1488, XP055416468, US ISSN: 0022-1767, DOI: 10.4049/jimmunol.1401127
- WIESNER ET AL.: 'Conditional Immortalization of Human B Cells by CD 40 Ligation' PLOS ONE vol. 3, no. 1, 23 January 2008, page E1464, XP055130901

## Description

### CROSS-REFERENCE TO RELATED PATENT APPLICATIONS

The present application claims the benefit of priority to United States Provisional Patent Application No. 62/295,728, filed on February 16, 2016.

### SEQUENCE LISTING

A Sequence Listing accompanies this application and was filed with the application as a text file.

### TECHNICAL FIELD

The invention relates to a method for producing and supporting B cells, including human B cells, which can be used to produce antigen-specific antibodies, and more particularly, monoclonal antibodies. More specifically, the invention relates to culturing B cells to produce monoclonal antibodies without using hybridoma technology or EBV-transformed B cells.

### BACKGROUND ART

Monoclonal antibodies have found particular utility in medicine, such as through the development of antibody-based biologicals or pharmaceuticals. Current methods for monoclonal antibody production involve the mouse hybridoma method; i.e., fusing antibody producing cells with myeloma cells. Other methods include EBV-transformed B cell lines and phage display. Each of the different methods used to produce human monoclonal antibodies suffers from technical limitations that render it difficult to use. For example, with hybridoma technology there is a need to "humanize" the antibody so as to reduce the frequency of promoting an immune response to nonhuman portions of the monoclonal antibody. Additionally, immune responses to a particular antigen or epitope may be species-specific. Other methods have drawbacks that limit their wide application for use in developing monoclonal antibodies for use in therapy and diagnosis for humans.

EP 2 495 312 A1 provides a method for producing an antigen-specific B cell population comprising IgG-positive B cells specific to a specific antigen. Masahiro Kitabatke et al., J Immunol. 2015 Feb 15;194(4):1480-8 discloses B1a cells from G5PR transgenic mice that differentiate into IgM and IgG autoantibody-secreting plasmablasts.

### SUMMARY OF THE INVENTION

The invention is set out in the appended set of claims.

The disclosure is based on the discovery of a highly efficient method for expanding and differentiating B cells in culture that enables single cell cloning of B cells. In one aspect, the discovery relates to a highly efficient method for expanding and differentiating human B cells in culture that enables single cell cloning of human B cells and production of antibodies.

The disclosure is also based on the discovery of a highly efficient method for expanding and differentiating B cells in culture that enables single cell cloning of B cells (seeding a single B cell, and expanding that B cell into a clone of B cells) in amount and time period sufficient to generate monoclonal antibody in an amount that facilitates characterization of the monoclonal antibody produced. In one aspect of this method, human B cells are expanded and differentiated as single clones to produce human monoclonal antibody. In another aspect of these methods, the method does not require and/or no antigen is added to the expansion culture. In still another aspect, the antigen-specificity of the B cells is unknown. In still another aspect, the B cells are not exposed to antigen *in vitro.*

In another aspect of the disclosure, an *in vitro* method for the production of monoclonal antibodies, particularly of the IgG type, is provided.

In another aspect of the disclosure, provided are feeder cell lines, comprising modified mesenchymal stromal cells or modified thymic epithelial cells, which can efficiently promote and support high numbers of B cells in culture, thereby allowing higher levels of monoclonal antibody production than that observed in prior attempts of culturing B cells with modified mouse 3T3 cells. Related to this aspect, the feeder cell lines comprising mesenchymal stromal cells or thymic epithelial cells, modified to express a combination comprising CD154 (also known as CD40L or CD40 Ligand) and BLyS (B Lymphocyte Stimulator, also known as BAFF (B-cell activating factor)), or a combination comprising CD154, BLyS, and IL-21. The feeder cells of the disclosure support significant proliferation and differentiation of B cells of mammalian species, including human B cells and murine B cells, without the need to add (i.e., in the absence of) exogenous antigen or exogenous IL-4 when B cells are cultured in the presence of the modified feeder cells *in vitro.*

In one aspect, provided is a method for producing monoclonal antibodies that bind to a particular antigen, typically a known antigen. The method includes the steps of isolating the B cells that are either naïve to antigen, or have been exposed to an antigen (e.g., either by *in vitro* or *in vivo* exposure); separating the isolated B cells into single cells; culturing an isolated B cell (single B cell) in the presence of modified feeder cells (i.e. feeder cells expressing CD154 or another CD40 agonist and BLyS or a comparable B cell activating factor) according to the disclosure *in vitro* under conditions and for a sufficient time to achieve at least an average of 10⁴-fold expansion in number (e.g., expansion from a single cell to 10,000 cells, average taken over wells of a multiwell plate, such as a 96 well plate) in less than 2 weeks in culture. A monoclonal antibody is produced from the expanded B cell clone in culture. Further steps may comprise characterization of the monoclonal antibody such as one or more of (a) determining the antigen specificity of the monoclonal antibody using methods known in the art; and (b) isolating the monoclonal antibody comprising (i) purification of the monoclonal antibody from the culture medium using methods known in the art, and/or (ii) isolating the total RNA from the B cell clone to produce cDNA encoding the variable-heavy (VH) antibody chains and cDNA encoding variable-light (VL) antibody chains which then can be cloned into a eukaryotic expression vector for recombinant production of the monoclonal antibody using methods known in the art. Alternatively, the selected B cell clones producing antigen-specific monoclonal antibodies can be immortalized by hybridoma technology, EBV-transformation, immortalized using the amphotropic retrovirus LXSN16E6E7 produced by the PA317 LXSN 16E6E7 cell line (American Type Culture Collection, CRL-2203^{™}), or other methods known in the art.

Another aspect of the disclosure provides a kit for expanding and differentiating mammalian B cells in culture. The kit may also be used for producing monoclonal antibodies from B cells expanded using the kit. The kit comprises modified feeder cells of the disclosure, reagents for isolating B cells from a biological sample, and packaging for holding the modified feeder cells and for holding the reagents. The kit may further comprise one or more of reagents necessary for culturing the modified feeder cells and B cells, and characterization of monoclonal antibodies produced from B cells co-cultured with the modified feeder cells using the kit.

### BRIEF DESCRIPTION OF THE FIGURES

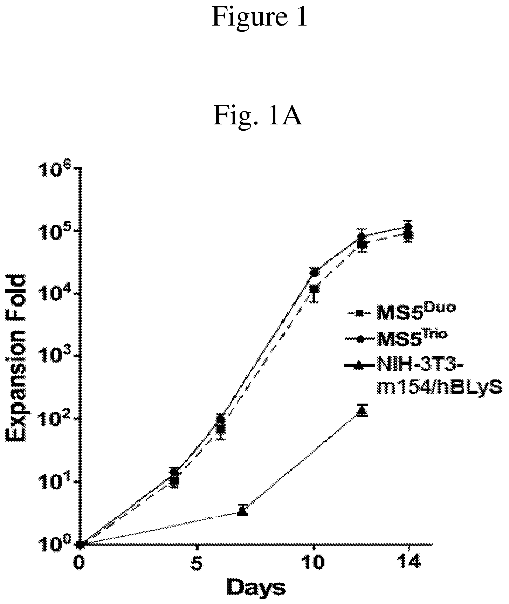

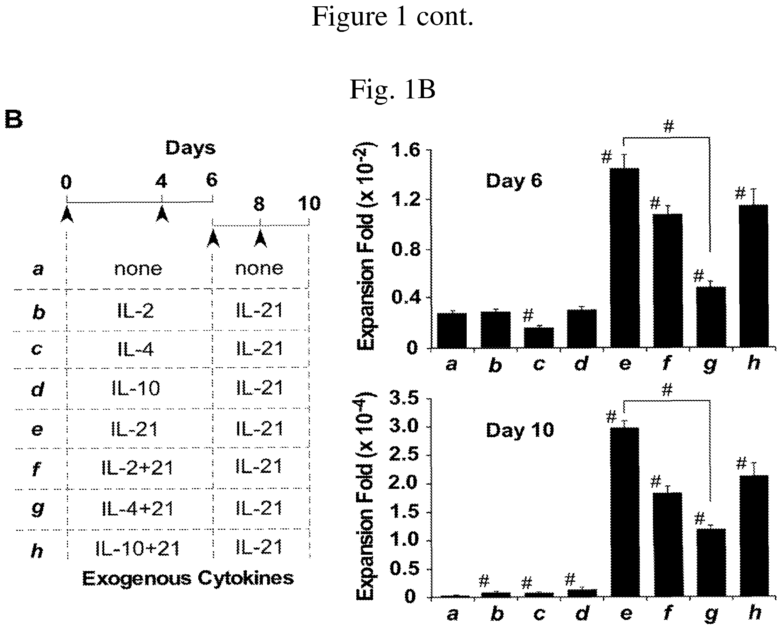

Figure 1 is a set of graphs showing B cell expansion. Fig. 1A is a graph showing human B cell expansion, as measured by fold expansion relative to the number of days of culture, as a result of being cultured with either modified feeder cells of the disclosure comprising stromal cells expressing human BLyS and CD154 (with added IL-21) ("MS5^{DUO}"), stromal cells expressing BLyS, CD154, and IL-21 ("MS5^{TRIO}") as compared to feeder cells comprising NIH-3T3 cells expressing BLyS and CD154 (with added IL-21)("NIH-313-m154/hBLyS). These results are representative of those obtained in ≥ 3 experiments.
Fig. 1B is a graph showing the effects of exogenous human cytokines on human B cell expansion. Values represent mean B cell numbers from duplicate 6 and 10 day cultures on MS5^{Duo} cell monolayers where the indicated cytokine combinations were present during the designated periods of time (a-h). All cytokines were present at 20 ng/mL except for IL-4 (10 ng/mL). Additional medium containing the appropriate cytokine(s) was added to the cultures on days 4 and 8. Values represent means (+ s.e.m.) of 6 samples from two independent experiments. Mean values significantly different between (a) and other cultures or between cultures (e) and (g) are indicated; ^{#}p < 0.05.

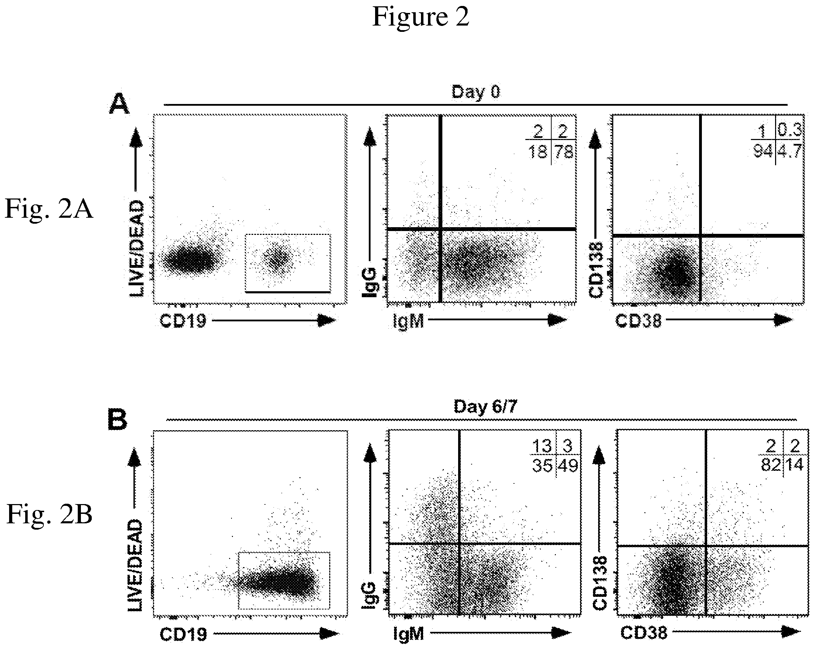

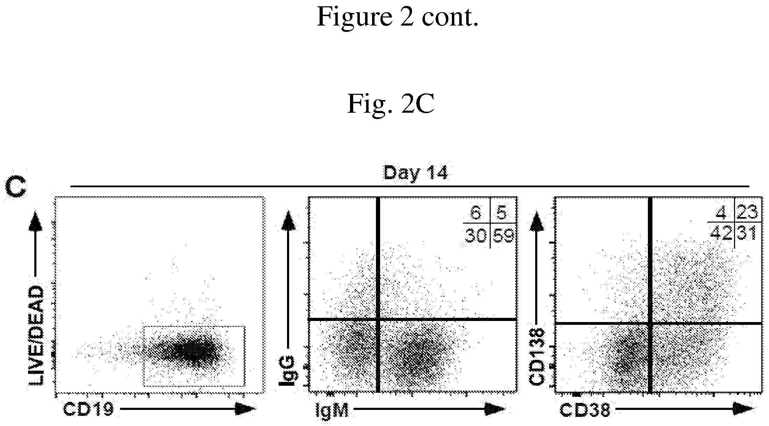

Figure 2 is a set of FACS histograms showing the levels of the indicated cell surface markers. Fig. 2A is a graph showing cell surface phenotype (IgM, IgG, CD38, and CD138) of CD19⁺ human B cells after isolation from peripheral blood but prior to expansion in the method of the disclosure. Mean frequencies of viable CD20⁺ or CD19⁺ B cells expressing IgM, IgG, CD38 or CD138 within the indicated quadrants are indicated from two independent experiments with similar results.
Fig. 2B is a graph showing cell surface phenotype (IgM, IgG, CD38, and CD138) of human B cells during ex vivo expansion as a result of being cultured on a monolayer of MS5^{Trio} cells after 6-7 days. These results are representative of those obtained in ≥ 3 experiments. Mean frequencies of viable CD20⁺ or CD19⁺ B cells expressing IgM, IgG, CD38 or CD138 within the indicated quadrants are indicated from two independent experiments with similar results.
Fig. 2C is a graph showing cell surface phenotype (IgM, IgG, CD38, and CD138) of human B cells during ex vivo expansion as a result of being cultured on a monolayer of MS5^{Trio} cells at 14 days of culture. Mean frequencies of viable CD20⁺ or CD19⁺ B cells expressing IgM, IgG, CD38 or CD138 within the indicated quadrants are indicated from two independent experiments with similar results.

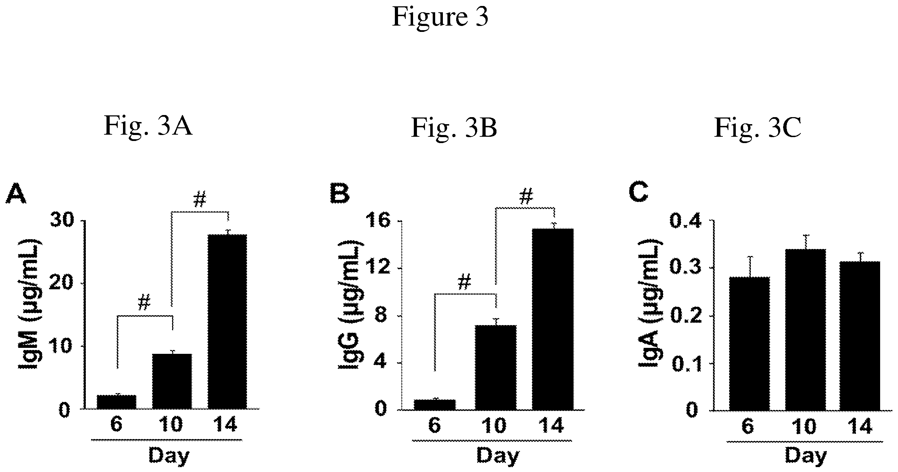

Figure 3 is a set of graphs showing antibody production by isotype. Fig. 3A is a graph showing IgM production in the tissue culture supernatant fluid of human B cells during ex vivo expansion as a result of being cultured on monolayer of MS5^{Trio} cells at days 6, 10, & 14 of culture. Bars represent means (+ s.e.m.) antibody concentrations in 4 samples from two independent experiments as determined by ELISA.
Fig. 3B is a graph showing IgG production in the tissue culture supernatant fluid of human B cells during ex vivo expansion as a result of being cultured on monolayer of MS5^{Trio} cells at days 6, 10, & 14 of culture. Bars represent means (+ s.e.m.) antibody concentrations in 4 samples from two independent experiments as determined by ELISA.
Fig. 3C is a graph showing IgA production in the tissue culture supernatant fluid of human B cells during ex vivo expansion as a result of being cultured on monolayer of MS5^{Trio} cells at days 6, 10, & 14 of culture. Bars represent means (+ s.e.m.) antibody concentrations in 4 samples from two independent experiments as determined by ELISA.

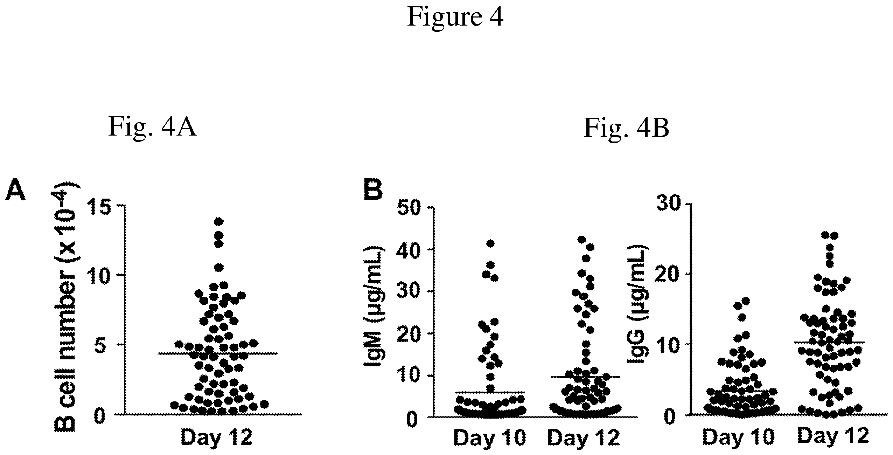

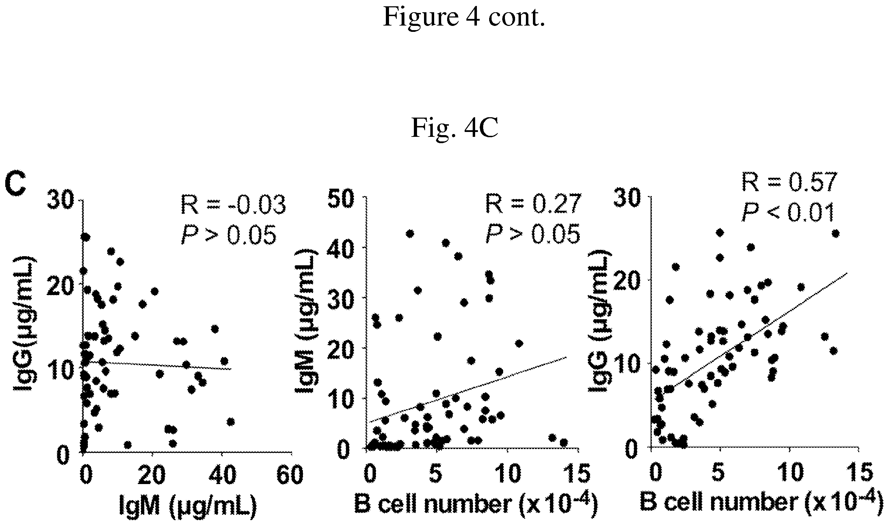

Figure 4 is a set of graphs showing the expanded cells and production of various isotypes of antibodies. Fig. 4A is a graph representing single human B cell clones (each dot representing an individual clone) and their expansion in number relative to day 12 of culture with a monolayer of MS5^{Trio} cells. These results are representative from two independent experiments.
Fig. 4B is a graph depicting concentrations of IgM and IgG in tissue culture supernatant fluid of the human B cell clones shown in Fig. 4A, as measured by ELISA, at days 10 and 12 of culture. These results are representative from two independent experiments.
Fig. 4C is a graph depicting the correlation between concentrations of IgM and IgG in tissue culture supernatant fluid of the human B cell clones shown in Fig. 4A, as measured by ELISA, on day 12, or those between single B cell clonal expansion and IgM or IgG (n=69). These results are representative from two independent experiments.

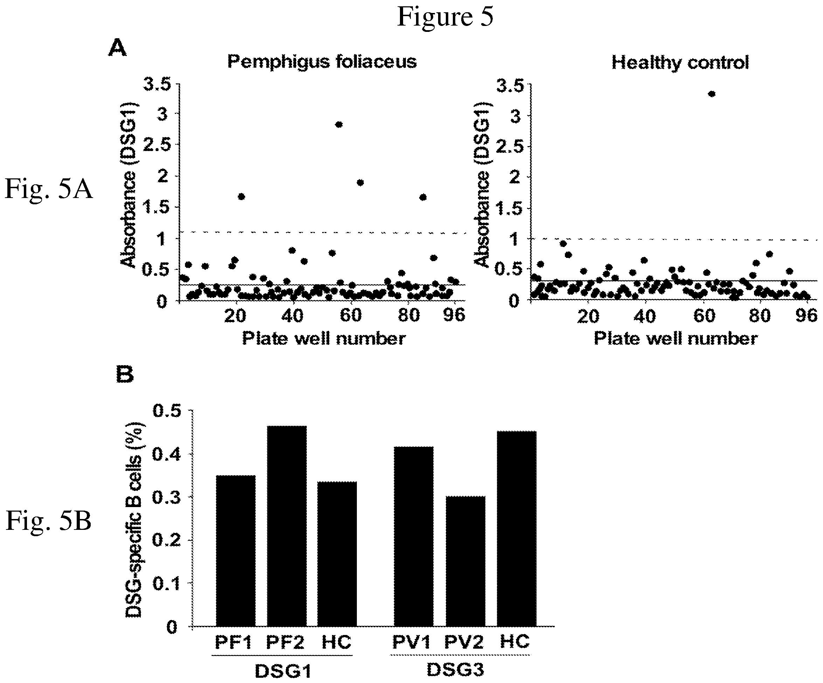

Figure 5 is a set of graphs showing production of specific antibody. Fig. 5A is a representative graph showing measurement of anti-desmoglein 1 (DSG1) IgG antibody by ELISA ("Absorbance") in relation to the well numbers of a 96 well plate ("Plate well number") containing isolated B cells, from a pemphigus foliaceus patient or a healthy control, and co-cultured with MS5^{Trio} cells. The solid horizontal line shows the mean absorbance value for all 96 wells of the plate. The dashed horizontal line indicates the mean plus 2 standard deviations value for all 96 wells of the plate. A well was considered positive when its absorbance value exceeded the dashed line.
Fig. 5B is a bar graph showing the frequency of antigen-specific B cells (having specificity for DSG1 or desmoglein 3 (DSG3)) as calculated from circulating B cells from two individuals with pemphigus vulgaris (PV1 and PV2) or two individuals with pemphigus foliaceus (PF1 and PF2), as compared to circulating B cells from a healthy individual without measurable anti-DSG serum autoantibodies ("Healthy control", HC). Values shown represent the mean frequency of wells that were positive for DSG1- or DSG3-specific IgG antibody for each individual. Eleven individual 96 well ELISA plates were assessed in each assay for each individual.

### DETAILED DESCRIPTION OF THE INVENTION

The disclosure herein is based on the discovery of an efficient method for expanding and differentiating B cells in culture, from single cell clones to an amount of B cells that produce sufficient quantities of monoclonal antibody enabling further characterization without relying on first cloning and expression of the immunoglobulin genes. Further characterization may comprise characterization of one or more of antigen specificity, function, binding, and or neutralization using one or more high throughput screening assays known in the art. The disclosure overcomes the deficiencies of feeder cell lines that were used previously to expand and differentiate murine B cells, such as the NIH-3T3 cell line (murine fibroblast cell line), in expanding human B cells in culture, particularly in an amount and state of differentiation to be useful in production of monoclonal antibodies.

Definitions- While the following terms are believed to be well understood by one of ordinary skill in the art of biotechnology, the following definitions are set forth to facilitate explanation of the invention.

The term "B cell" is used herein to mean a mammalian B lymphocyte including, but not limited to, human B cell, and murine B cell. Any B cell may be expanded in a method of the present disclosure, and for methods of the disclosure involving antibody production, such as (a) naive B cells, which comprise a diverse antibody repertoire generated by DNA rearrangements during B cell development, and (b) B cells that have been exposed to, or have memory of the exposure to, a specific antigen. B cells include B cell populations, subpopulations, or subsets thereof, including but not limited to, naïve B cells, memory B cells, activated B cells, B1 cells, germinal center B cells, marginal zone B cells, regulatory B cells, and follicular B cells.

The term "monoclonal antibody" is used herein to mean an antibody that displays a single binding specificity and affinity for an epitope of an antigen. "Recombinant monoclonal antibodies" is a term that refers to monoclonal antibodies that are produced by recombinant means, such as by using a recombinant expression vector containing immunoglobulin gene sequences (e.g., VH and VL cDNA) and transfected into a host cell. Such a recombinant expression vector may comprise human immunoglobulin gene sequences (e.g., human VH and human VL cDNA), which are then transfected into a host cell. Recombinant monoclonal antibodies also comprise murine VH and VL cDNA, which are engineered to produce "humanized" antibodies that can be used for therapeutic, diagnostic, or theranostic applications in humans.

The term "antigen" is used herein to mean a substance that can stimulate the production of antibody, and can be bound by antibody specific for the substance (i.e., an antibody can specifically bind an antigen for which it has binding specificity). Antigens may be comprised of a substance comprising one or more of protein, peptide, lipid, carbohydrate, nucleic acid, and small molecule (organic or inorganic). Antigens may include: a substance foreign to the human body, viral antigens, bacterial antigens, parasite antigens, tumor antigens, toxin antigens, fungal antigens, self antigens, altered self antigens (self antigens that are altered or modified as the result of a disease state), modified antigens (misfolded or oxidized or with altered glycosylation or overexpression or mutated, as a result of a disease state and as compared to the antigen in a healthy or non-disease state).

The term "modified feeder cell" and "feeder cell" are used herein to mean mesenchymal stromal cells or thymic epithelial cells, modified to express a combination comprising a CD40 agonists such as CD154 (also known as CD40L or CD40 Ligand) and a B cell survival promoter such as BLyS (B Lymphocyte Stimulator, also known as BAFF (B-cell activating factor), or a combination comprising CD154, BLyS, and IL-21. Specific examples of such modified feeder cells are modified mesenchymal stromal cell lines (e.g., MS-5 cells available as catalog number ACC 441 from Deutsche Sammlung von Mikroorganismen und Zellkulturen (DSMZ), Braunschweig, Germany) and modified thymic epithelial cell lines (e.g., TEC cells). As an illustrative example, MS-5 is a murine mesenchymal stromal cell line that is commercially available. It is noted that mesenchymal stromal cell lines are distinguished from fibroblast cell lines, such as the NIH-3T3 cell line (murine fibroblast cell line NIH/3T3, deposited as ATCC CRL-1658), in that mesenchymal stromal cell lines secrete different factors and can support different functions (including vasculogenesis, angiogenesis) than fibroblast cell lines. For example, MS-5 cells secrete significantly higher levels of CXCL12 (SDF-1), CXCL-16, Angiopoetin-1, MCP-1 (monocyte chemoattractant protein-1, also known as CCL2), NOV (nephroblastoma overexpressed, also known as CCN3) and HGF (hepatocyte growth factor) than NIH 3T3 cells (Zhou et al., 2012, Br. J. Haematology, 157:297-311). TEC or thymic epithelial cell lines (e.g., TEC-84, TEC-D11) support human lymphopoiesis (Beaudette-Ziatanovo, Exp. Hematol., 39(5): 570-579). Thymic epithelial cell lines can be induced to undergo biological changes to assume a mesenchymal cell phenotype (epithelial-mesenchymal transition) such as by treatment with transforming growth factor in culture. The mesenchymal stromal cells or thymic epithelial cells are engineered to express a combination comprising a CD40 agonist such as a CD154 polypeptide and a B cell survival promoter such as BLyS, or a combination comprising these two polypeptides and IL-21, in forming the modified feeder cells of the disclosure. If the feeder cells are not engineered to express IL-21, then IL-21 must be added exogenously to the culture medium during the culture period. The culture period is suitably less than 3 weeks, suitably less than 2 weeks. The culture period may be 5, 6, 7, 8, 9, 10, 11, 12 or more days.

BLys may be expressed on the surface or may be soluble after cleaved from the cell surface. Alternatively the BLyS is replaced by a different factor(s) that promotes B cell survival in culture including BLyS fragments, APRIL, CD22 ligand, CD22 monoclonal antibody, or fragments thereof. BLyS is a 285 amino acid glycoprotein (SEQ ID NO: 69) encoded by a nucleic acid sequence comprising SEQ ID NO:01 or a similar nucleotide sequence encoding the same amino acids. BLyS, produced recombinantly by the modified feeder cells of the disclosure, may vary in length as long as it can bind and signal the BLyS receptor, and promote expansion of human B cells. In that regard, BLyS can be produced as a membrane bound type, or in soluble form. For example, a soluble form comprised of amino acids 136-285 has been found to be functionally active.

The CD154 polypeptide may be replaced by any CD40 agonist including but not limited to CD40 antibodies and fragments thereof, the CD40 ligand, CD154 polypeptide and polypeptide fragments thereof, small molecules, synthetic drugs, peptides (including cyclic peptides), polypeptides, proteins, nucleic acids, aptamers, synthetic or natural inorganic molecules, mimetic agents, and synthetic or natural organic molecules capable of activating CD40. In a certain embodiment, the CD40 agonist is a CD40 antibody. The CD40 antibodies can be of any form. Antibodies to CD40 are known in the art (*see, e.g.,* Buhtoiarov et al., 2005, J. Immunol. 174:6013-22; Francisco et al., 2000, Cancer Res. 60:3225-31; Schwulst et al., 2006, 177:557-65). The CD40 agonists may be CD154 polypeptides and may be expressed on the surface of feeder cells or expressed in soluble form. Human CD154 polypeptide was used in the Examples and can be found as a 261 amino membrane bound protein (SEQ ID NO: 68) or 149 amino acid soluble protein, and can be encoded by a nucleotide sequence comprising SEQ ID NO:02 or SEQ ID NO:03, or a similar nucleotide sequence encoding the same amino acids. In that regard, CD154 can be produced as a membrane bound type, or in soluble form, of varying lengths. It has been shown that amino acids important for CD40L and CD40 interactions include amino acids 128 to 258.

Human IL-21 (mature polypeptide) comprises 131 amino acids (SEQ ID NO: 70), and can be encoded by a nucleotide sequence comprising SEQ ID NO:04, or a similar nucleotide sequence encoding the same amino acids. In that regard, IL-21 can be produced in various lengths as long as binding and induction of signaling through the IL-21 receptor is maintained, and it has been shown that amino acids important for IL-21 binding and functional interactions include, but are not limited to, amino acids 33, 145, and 148. The feeder cells can be engineered to express IL-21 or the IL-21 can be added to the culture exogenously. The IL-21 can be added at between 2ng/mL and 1000 ng/mL, suitably between 5 and 500 ng/mL, suitably between 10 and 100 ng/mL.

The term "exposed to an antigen" is used herein to mean that the antigen is contacted with a B cell in sufficient concentration and for a sufficient time for the B cell to become activated by the antigen (e.g., binds to B cell receptors (BCR), or to induce B cell differentiation into B cells capable of producing antibody specific for the triggering antigen ("specific antigen"). There are a number of ways by which B cells may be exposed to antigen *in vivo* including, but not limited to, infection, injection, vaccination, immunization, and circulation (e.g., tumor antigen, altered self antigen, self antigen). In another embodiment, B cells may be exposed to and activated by a specific antigen in *vitro.* For example, US published patent application US2015/0299655 discloses a method for *in vitro* immunization of human B cells by a specific (e.g., known) antigen comprising culturing a total population of human peripheral blood mononuclear cells in the presence of an antigenic composition comprising at least one known antigen that is coupled to a Tat protein and a ligand to a receptor of an antigen-presenting cell (the latter including saccharides that bind C-lectin type receptors, immunoglobulins or fragments thereof (e.g., containing Fc portion) that bind Fc receptors) for sufficient time and under sufficient conditions for B cells present in the human peripheral blood mononuclear cells to be immunized by the antigenic composition.

B cells, exposed to antigen *in vivo,* may be isolated from a biological sample comprising peripheral blood or other body fluids (e.g., synovial fluid, or exudates) or from tissues (e.g., any tissue from the body of an individual, including but not limited to bone marrow, cardiac tissue, nervous tissue, tumor tissue, diseased tissue, connective tissue, spleen tissue, lymph node tissue, connective tissue, thymus tissue, and other lymphoid tissues) using methods known in the art. Such methods include fractionation using antibody coated magnetic beads and fluorescence-activated cell sorting. In immunomagnetic sorting, positive and/or negative selections may be performed to isolate the B cells. Reagents for isolating B cells include one or more antibody preparations for isolating B cells. Antibodies which are specific for (can bind to) cell surface markers on B cells include antibodies specific for IgM, IgD, IgG, IgA, IgE, CD19, CD20, CD21, CD22, CD24, CD40, CD72, CD79a, CD79b, or combinations (particularly for isolating B cell subpopulations or subsets) of these with, or combinations including, additional cell surface molecules such as CD5, CD9, CD10, CD23, CD38, CD48, CD80, CD86, CD138 or CD148. Antibodies or an antibody combination may be bound to magnetic beads in forming reagents for isolating B cells by immunomagnetic separation or sorting. The antibodies may be bound to a fluorescent label, as well known in the art, to form reagents for isolating B cells by fluorescence-activated cell sorting. Isolating B cells for seeding single B cell cultures can be done using methods known in the art which include, but are not limited to, limiting dilution or dilution cloning, and fluorescence activated cell sorting.

The methods of expanding B cells in vitro or ex vivo provided herein include isolating at least one B cell from a subject. The B cells used in the methods may be harvested from various areas of the subject, including but not limited to the blood, spleen, peritoneal cavity, lymph nodes, bone marrow, site of autoimmune disease, site of inflammation or tissue undergoing transplant rejection in the subject. The cells may be harvested from the subject by any means available to those of skill in the art. The harvested population of cells should contain B cells, but may be a mixed cellular population. The subject may be any animal with B lymphocytes, suitably a mammal, suitably a domesticated animal such as a horse, cow, pig, cat, dog, or chicken, or suitably a human. Alternatively, the cells may be derived from stem cells, including but not limited to B cell stem cells, bone marrow stem cells, embryonic stem cells and induced pluripotent stem cells, which have been appropriately differentiated *in vitro* to develop into B cells or B cell progenitors prior to use in the methods described herein. See e.g., Carpenter et al., 2011, Blood 117: 4008-4011. The B cells may be naïve or antigen-exposed cells.

The B cells may be isolated using the isolation and selection methods described above or other methods known to those of skill in the art and may include both positive and negative selection steps. The B cells may be less than 100% B cells. Isolating is used to indicate that a group of cells is separated from incubation media, feeder cells or other non-B cells. Isolating is not meant to convey that the resulting isolated cells have a certain level of purity or homogeneity. The cells may be harvested, isolated or selected using any means available to those of skill in the art. For example, B cells may be harvested from adherent cells by selecting for non-adherent cells after an appropriate incubation. Cells may also be selected for expression of cell surface markers by FACS sorting or by the differential ability to bind antibody coated magnetic beads. Means of selecting cells in a mixed population are well known to those skilled in the art.

The isolated B cells are then cultured with a feeder cell line comprising a stromal cell line modified to express a CD40 agonist and a B cell survival promoter and optionally IL-21. The IL-21 may be added exogenously to the culture media. The culturing step is carried out without adding additional exogenous IL-4. The term "without additional exogenous IL-4" indicates that the culture comprises less than 200pg/mL IL-4, less than 100pg/mL IL-4, less than 50pg/mL IL-4 or suitably less than 5pg/mL IL-4 added exogenously to the culture. This is distinct from the mouse B cell expansion methods reported by either U.S. Patent No. 8,815,543 or U.S. Publication Number 2014/0065118. As used herein expansion of B cells includes stimulation of proliferation of the cells as well as prevention of apoptosis or other death of the cells. As used herein, "culturing" and "incubation" are used to indicate that the cells are maintained in cell culture medium at 37°C and 5% CO₂ for a period of time with the indicated additives (feeder cells, cytokines, agonists, other stimulatory molecules or media, which may include buffers, salts, sugars, serum or various other constituents). Suitably, the incubation or culturing periods used herein is at least 48 hours, but may be for any amount of time up to eight or more days. Those of skill in the art will appreciate that the culturing or incubation time may be varied to allow proper expansion, to adjust for different cell densities or frequencies of individual subsets, and to allow an investigator to properly time use of the cells. Thus the precise culture length may be determined empirically by one of skill in the art. The culturing period may also be carried out without addition of any exogenous cytokines if the feeder cells are modified to express Il-21 or Il-21 may be the only exogenous cytokine added to the cultures.

The methods may allow from two fold to over 5 × 10⁶ fold expansion of B cells. The B cells are expanded at least an average of 10³, 10⁴, or 10⁵ fold in less than 2 weeks in culture. The cells may be selected after the culture period to remove any non-B cells or to positively select for B cells or for a particular B cell subset. The culturing step may include an antigen or may be completed without addition of an antigen. Without addition of an antigen is similar to without addition of IL-4 as defined above. The expanded B cells may have undergone class-switching during the culturing period. In one embodiment, the isolated B cells were positive for IgM and after expansion in the methods described herein at least 10%, 15%, 20%, 25% of the expanded B cells express IgG. In one embodiment, at least 1%, 2%, 3%, 4%, 5%, 7%, 10% or more of the expanded B cells express IgA. If the cells are separated into single B cells prior to the culturing step the resulting expanded B cells may be used to produce and isolate a monoclonal antibody. The monoclonal antigen specificity can be determined using methods available to those of skill in the art and the monoclonal antibodies can be purified from the B cells.

The present disclosure is not limited to the specific details of construction, arrangement of components, or method steps set forth herein. The compositions and methods disclosed herein are capable of being made, practiced, used, carried out and/or formed in various ways that will be apparent to one of skill in the art in light of the disclosure that follows. The phraseology and terminology used herein is for the purpose of description only and should not be regarded as limiting to the scope of the claims. Ordinal indicators, such as first, second, and third, as used in the description and the claims to refer to various structures or method steps, are not meant to be construed to indicate any specific structures or steps, or any particular order or configuration to such structures or steps. All methods described herein can be performed in any suitable order unless otherwise indicated herein or otherwise clearly contradicted by context. The use of any and all examples, or exemplary language (e.g., "such as") provided herein, is intended merely to facilitate the disclosure and does not imply any limitation on the scope of the disclosure unless otherwise claimed. No language in the specification, and no structures shown in the drawings, should be construed as indicating that any non-claimed element is essential to the practice of the disclosed subject matter. The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

Recitation of ranges of values herein are merely intended to serve as a shorthand method of referring individually to each separate value falling within the range, unless otherwise indicated herein, and each separate value is incorporated into the specification as if it were individually recited herein. For example, if a concentration range is stated as 1% to 50%, it is intended that values such as 2% to 40%, 10% to 30%, or 1% to 3%, etc., are expressly enumerated in this specification. These are only examples of what is specifically intended, and all possible combinations of numerical values between and including the lowest value and the highest value enumerated are to be considered to be expressly stated in this disclosure. Use of the word "about" to describe a particular recited amount or range of amounts is meant to indicate that values very near to the recited amount are included in that amount, such as values that could or naturally would be accounted for due to manufacturing tolerances, instrument and human error in forming measurements, and the like. All percentages referring to amounts are by weight unless indicated otherwise.

No admission is made that any reference, including any non-patent or patent document cited in this specification, constitutes prior art. In particular, it will be understood that, unless otherwise stated, reference to any document herein does not constitute an admission that any of these documents forms part of the common general knowledge in the art in the United States or in any other country. Any discussion of the references states what their authors assert, and the applicant reserves the right to challenge the accuracy and pertinence of any of the documents cited herein. The present disclosure shall control in the event there are any disparities between any definitions and/or description found in the cited references.

Unless otherwise specified or indicated by context, the terms "a", "an", and "the" mean "one or more." For example, "a protein" or "an RNA" should be interpreted to mean "one or more proteins" or "one or more RNAs," respectively.

The following examples are meant only to be illustrative and are not meant as limitations on the scope of the invention or of the appended claims.

### EXAMPLE 1

In this example, provided is a method for the *in vitro* expansion of human B cells according to the disclosure. Heparinized blood was collected from healthy adult human donors. Blood mononuclear cells were isolated by centrifugation over a layer of ficoll-sodium metrizoate in a Sepmate50 tube at 300 x g for 10 minutes. Peripheral blood mononuclear cells (PBMCs) were diluted 2-fold with buffer (PBS), and then centrifuged (300 x g, 10 min) and resuspended to ^{~}3 × 10⁷ cells/mL in MACS buffer (0.5% bovine serum albumin (BSA, w/v), 2.5 mM ETDA in PBS). CD19⁺ B cells were isolated by positive selection using CD19 magnetic beads according to the manufacturer's instructions. Briefly, isolated mononuclear cells were incubated with CD19 monoclonal antibodycoated microbeads (60 µL microbeads/mL of PBMCs) at 4 °C for 15 minutes, diluted 10-fold, pelleted again by centrifugation, and resuspended in 1 mL MACS buffer. The cells were loaded on a prewetted LS column (a column comprising a matrix composed of ferromagnetic spheres, which are covered with a coating allowing fast and gentle separation of cells) under a magnetic field. The column was then washed with an additional 6 mL of MACS buffer. CD19⁺ cells were eluted by removing the column from the magnetic field and adding 3 mL of RPMI 1640 medium containing 10% FCS (fetal calf serum) to the column and collecting the eluate in a separate conical tube. Viable cell concentrations were determined using a hemocytometer under a microscope with Trypan Blue staining of dead cells. B cell purities were determined by immunofluorescence staining and flow cytometric analysis with CD19⁺ cell frequencies of ≥98%. Additional rounds of selection can be used to enhance purification.

Expanding human B cells *in vitro* was first attempted using culture conditions that induced murine B cell expansion. Purified human B cells were cultured on stromal cell monolayers comprising NIH-3T3 cells expressing BLyS and CD154 (NIH-3T3-m154/hBLyS) with added human IL-21 under culture conditions mimicking mouse B cell cultures (patent application US20140065118). Expanding purified human B cells *in vitro* was subsequently attempted using 3T3-CD154^{EAT} or 3T3-CD154^{BEAT} cells under culture conditions mimicking mouse B cell cultures in the presence of exogenous recombinant cytokine mixtures, including IL-2, IL-4, IL-21, IL-10 and APRIL. Briefly, NIH-3T3-m154/hBLyS cells were supertransfected with cDNAs encoding mouse CD154 and mouse BLyS to generate NIH-3T3-CD154^{EAT} cells, or cDNAs encoding mouse CD154, mouse BLyS and mouse IL-21 to generate NIH-3T3-CD154^{BEAT} cells. Stromal cell lines expressing these cDNAs were isolated, subcloned and assayed for their ability to support mouse and human B cell expansion. Stromal cells were seeded in 24-well plates at a density of 3 × 10⁵ cells/well in 1 mL of culture media for at least 12 hours prior to the addition of purified human B cells (3 × 10³/well) to the cultures. When human B cells were added to the cultures, the media from each well was replaced with fresh culture media containing IL-4 (10 ng/mL). After 4 days of culture, additional media (0.7 mL) containing IL-4 (10 ng/mL) was added to each well. On day 6 of culture, the B cells and stromal cells were collected after treatment with 0.4 mL of trypsin-EDTA. The cell suspension was pelleted by centrifugation and resuspended in 2 mL of culture medium. B cell numbers were quantified by microscopy (hemocytometer), and the frequency of B cells was determined by immunofluorescence staining with flow cytometry analysis. However, while significant mouse B cell expansion was observed under these conditions, significant human B cell survival or expansion was not observed in these attempts (e.g., expansion was less than 130 fold). Therefore, NIH-3T3 cells were transfected with human BLyS and human CD154 cDNAs, with CD154⁺BLyS⁺ cells isolated, expanded and subsequently subcloned. Under these conditions, maximal human B cell expansion within a 9 to 12 day culture period was not increased significantly beyond that observed using NIH-3T3-mCD154/hBLyS cell monolayers (patent application US20140065118) as human B cell expansion within a 12 day period was only 130 ± 17-fold (mean ± s.e.m.; Fig. 1A). Human B cell expansion was not significantly improved by the addition of exogenous human recombinant cytokine mixtures, including IL-2, IL-4, IL-21, IL-10 and APRIL. Therefore, a series of additional human and mouse stromal cells were assessed for their ability to support human B cell expansion.

Diverse human stromal cells and mouse stromal cells were transfected with human BLyS and human CD154 using the following vectors and methods of clonal selection. cDNA encoding human BLyS was used to generate a BLyS-IRES-eGFP DNA construct in order to provide a (GFP - green fluorescent protein) selection marker independent of immunofluorescence staining for CD154 expression. Human CD154 cDNA and human BLyS-IRES-eGFP DNA were independently cloned into a retroviral-based expression vector having LTR as the promoter (pMSCVpuro vectors) and transfected into the stromal cell lines by retroviral transduction using a commercially available retroviral packaging cell line. After transfection, stable puromycin (5 µg/mL)-resistant cells were subsequently selected during culture. Cells expressing CD154 and secreting BLyS were visualized by staining the cell surface of stromal cells with APC (Allophycocyanin)-conjugated CD154 monoclonal antibody, with APC⁺GFP⁺ (CD154⁺BLyS⁺) cells isolated using fluorescence-activated cell sorting. Isolated CD154⁺BLyS⁺ cells were expanded in culture, subcloned and tested for their ability to support and expand human B cells in vitro. Stromal cells tested for their ability to support and expand human B cells included: mouse MS-5 cells, a bone marrow stromal cell line; AFT024 cells, a mouse fetal liver stromal cell line; OP9 cells, a mouse bone marrow stromal cell line; human A549 cells, an adenocarcinomic alveolar basal epithelial cell line; human EA-Hy926 cells, a human endothelial cell line; HS-5 cells, a human fibroblastoid stromal cell line; TEC-84 cells, a human thymic stromal cell line; human foreskin fibroblasts immortalized using the amphotropic retrovirus LXSN16E6E7 produced by the PA317 LXSN 16E6E7 cell line (American Type Culture Collection, CRL-2203^{™}); and similarly-transformed human umbilical vein endothelial cells. The results showed that most stromal cell lines expressing human BLyS and CD154 were either unable to support B cell survival or expansion, or were less efficient in supporting expansion of human B cells than transfected mouse NIH-3T3 cells. Surprisingly, and unexpectedly, MS-5 cells and TEC-84 cells demonstrated an ability to support and expand human B cells in culture, with subsequent MS-5 cell clones having the most optimal growth characteristics for supporting human B cell expansion without a significant need to arrest stromal cell proliferation by treating the cells with mitomycin C to prevent cell division.

The MS-5 cells and TEC-84 cells, modified to express human CD154 and BLyS, were further and similarly modified to express IL-21. Human IL-21 cDNA was used to generate an IL-21-2A-peptide-mBFP2 DNA construct enabling expression of Blue Fluorescent Protein (mBFP2) via a cleavable 2A peptide sequence. IL-21-2A-peptide-mBFP2 DNA was inserted in place of the puromycin gene of the pMSCVpuro vector. Stable puromycin (5 µg/mL)-resistant cells were subsequently selected during culture. Modified feeder cells that were APC⁺GFP⁺BFP⁺ (CD154⁺BLyS⁺IL-21⁺) were isolated using fluorescence-activated cell sorting, and single cell clones of each stromal cell line were subsequently isolated and tested for their ability to support human B cell proliferation.

Modified feeder cells of the disclosure were compared with NIH-3T3 cells transfected with human BLyS and mouse CD154 cDNAs (NIH-3T3-mCD154/hBLyS) for the ability, as feeder cells, to support and induce expansion of human B cells isolated from blood. In this comparison, viable human CD19⁺ B cells were added to the respective cultures of fresh feeder cell monolayers. Human B cells were cultured on NIH-3T3-mCD154/hBLyS cell monolayers with exogenous human IL-4 (2 ng/ml) for 7 days. Additional medium containing IL-4 (2 ng/ml) was added to the cultures on days 2 and 4. B cells were isolated on day 7 and transferred onto fresh NIH-3T3-mCD154/hBLyS cell monolayers with exogenous human IL-21 (10 ng/ml) for 5 days. In cultures using MS-5 cells transfected with human CD154 and BLyS (MS5^{Duo} cells), B cells were cultured on MS5^{Duo} cells with the addition of IL-4 plus IL-21 for 6 days, then cultured with exogenous IL-21 until day 14. In cultures using MS-5 cells transfected with human CD154, BLyS and IL-21 (MS5^{Trio} cells), B cells were cultured on MS5^{Trio} cells with the addition of IL-4 for 6 days, then cultured without exogenous cytokines until day 14. On days 12 or 14, the B cells and stromal cells were collected from each well and analyzed. Fig. 1 shows a comparison of modified feeder cells comprising MS-5 clones that expressed human CD154 and BLyS (MS5^{Duo}) or MS-5 clones that expressed human CD154, BLyS and IL-21 (MS5^{Trio}) with NIH-3T3-mCD154/hBLyS cells, in relation to the expansion of human CD19⁺ B cells *in vitro* (expansion fold is relative to numbers of B cells present in the cultures at the initiation of the cultures). With MS5^{Duo} cells or MS5^{Trio} cells as the modified feeder cells, human B cells expanded by 12,018 ± 5,523-and 21,611 ± 3,576-fold at day 10, and 61,547 ± 16,134- and 80,761±28,979-fold by day 12, respectively (Fig. 1A). With NIH-3T3-mCD154/hBLyS feeder cells, human B cells expanded by 3.4-fold by day 7 and ^{~}130-fold by day 12. It is also noted that cultures of B cell with either MS5^{Duo} cells or MS5^{Trio} cells can be performed in the absence of IL-4 with similar or better results for amplification and differentiation (Figs. 1B and 2B-C). Thus, modified feeder cells of the disclosure can significantly promote expansion of human B cells as compared to NIH-3T3-mCD154/hBLyS cells as feeder cells. The modified feeder cells of the disclosure provide a significantly more optimal stromal cell monolayer for human B cell expansion in comparison with the majority of mouse and human cell lines that were tested for this capacity.

### EXAMPLE 2

Illustrated in this Example is the ability of the modified feeder cells of the invention to induce differentiation in human B cells expanded *in vitro.* Human B cells expanded *in vitro* in the MS5^{Duo} or MS5^{Trio} culture systems were analyzed for markers indicative of differentiation. Human B cell phenotypes were assessed using PerCP-, FITC-, PE/Cy7, FITC, and PE/Cy7-conjugated CD19 (HIB19), IgM (MHM-88), IgG (HP6017), CD38 (HIT2), and CD138 (MI15) monoclonal antibodies. Viable cells were analyzed by flow cytometry. Human B cells that expanded in the MS5^{Duo} or MS5^{Trio} culture systems remained CD19⁺ and CD20⁺, confirming their B cell origin (Fig. 2). Approximately eighty percent of freshly isolated human blood B cells expressed IgM, with ~4% expressing IgG prior to being cultured with the modified feeder cells (Fig. 2A). On day 7 of culture with a modified feeder cell monolayer, half of the B cells expressed IgM and ~16% expressed IgG (Fig. 2B). By the end of culture, most B cells expanded in the MS5^{Trio} culture system expressed cell surface IgM, but the frequency of IgM⁻IgG⁻IgA⁺CD19⁺ B cells had expanded (Fig. 2B-C). Less than 10% of freshly isolated B cells expressed CD38 or CD138 prior to being cultured with the modified feeder cells (Fig. 2A), which are activation markers expressed on activated B cells and expressed at high densities on plasma cells. By day 6-7 of culture, 16% of B cells expanded in the MS5^{Trio} culture system expressed the CD38 activation marker (Fig. 2B). By day 14 of culture, up to 56% of these B cells expressed CD38, with 23% of the B cells expressing both CD38 and CD138 (Fig. 2C). Similar, if not identical results were obtained with B cells expanded in the MS5^{Duo} culture system. Some of the human B cells cultured with modified feeder cells of the disclosure acquired a phenotype consistent with their activation and differentiation into antibody-secreting plasmablasts.

### EXAMPLE 3

Illustrated in this Example is the ability of the modified feeder cells of the disclosure to induce human B cells expanded *in vitro* to produce antibody. Human B cells expanded *in vitro* in the MS5^{Duo} or MS5^{Trio} culture systems were analyzed for the production of antibody. Human IgG, IgM, and IgA antibody levels were determined by enzyme-linked immunoassays (ELISA). Plates were blocked with tris-buffered saline containing 1% BSA before cell culture supernatant fluid (diluted 1:10 from bulk B cell cultures or undiluted if from single B cell cultures) was added to the plates. Alkaline-phosphatase-conjugated anti-IgG₁ antibody was used to detect bound antibody, and 1 M diethanolamine/0.5 M MgCl₂ with 4-nitrophenyl phosphate disodium salt hexahydrate was used as the detection reagent. Absorbance was read at 405 nm. Control plates were coated using 1% BSA in phosphate buffered saline (PBS), and blocked as above before the addition of culture supernatant fluid and detection reagents. Antibody concentrations were quantified based on standard curves obtained for each ELISA using commercially available human IgM, IgG and IgA standards.

Consistent with human B cell phenotypic changes during their *in vitro* expansion as described in Example 2 herein, secreted IgM was not detected in the tissue culture supernatant fluid of human B cells expanded *in vitro* in the MS5^{Trio} culture system by day 6 (Fig. 3A). However, IgM concentrations increased to 7.8 µg/mL by day 10, and 92 µg/mL on day 14. Similarly, IgG levels increased significantly to 2.4 µg/mL by day 14 (Fig. 3B). IgA levels also increased on days 10 and 14, but remained less than 0.3 µg/mL (Fig. 3C). Similar, if not identical results were obtained with B cells expanded in the MS5^{Duo} culture system. By contrast with human B cells expanded *in vitro* in the MS5^{Duo} or MS5^{Trio} culture systems, human B cells cultured on NIH-3T3-mCD154/hBLyS, NIH-3T3-CD154^{EAT}, or 3T3-CD154^{BEAT} stromal cell monolayers under similar conditions did not secrete measurable antibody into the tissue culture supernatant fluid. Thereby, as a result of expansion on the modified feeder cells of the disclosure, some human B cells differentiate during *ex vivo* expansion and predominantly secrete IgM and IgG antibodies.

In one aspect of the disclosure, provided is a method for producing human monoclonal antibodies that bind to a particular antigen, typically a known antigen. The method includes the steps of isolating human B cells, separating the isolated B cells into single cells, culturing an isolated B cell (single cell) in the presence of modified feeder cells according to the disclosure under conditions and for a sufficient time to achieve at least a 10⁴-fold expansion in number (e.g., expansion from a single cell to 10,000 cells, average expansion number over multiple wells of a multiwell plate) in less than 2 weeks in culture, wherein produced from the expanded B cell clone in culture is monoclonal antibody. This aspect excludes the addition of antigen, in attempts to guide antigen selection or further induce antigen-sensitization, directly to the coculture of isolated B cells with the modified feeder cells of the disclosure (i.e., exogenous antigen is absent when isolated B cells are cultured in the presence of modified feeder cells, as this is not necessary to produce monoclonal antibodies according to the method of the disclosure).

To illustrate this aspect, single human B cells were cultured on MS5^{Trio} stromal cell monolayers in 96-well plates without the addition of exogenous cytokines. In these limiting dilution assays, human B cell colonies were observed in 60 to 70% of wells; reflecting cloning efficiencies of 60% to 70%. In the wells containing B cell colonies, single B cells expanded 46,689±4,105-fold on average after 12 days (Fig. 4A). Tissue culture supernatant fluid IgM concentrations on days 10 and 12 of culture were 5.5 ± 1.2 µg/mL and 9.3 ± 1.4 µg/mL, respectively (Fig. 4B). IgG concentrations on days 10 and 12 of culture were 3.5 ± 0.5 µg/mL and 10.5 ± 0.8 µg/mL, respectively. Although there was no correlation between IgM and IgG secretion within individual B cell clones and no correlation between IgM secretion and B cell expansion, there was a significant positive correlation between IgG secretion and B cell expansion (Fig. 4C). Thus, some B cells within individual wells underwent isotype switching, with some of the B cells also differentiating into antibody-secreting cells. The significant concentrations of antibody produced for each B cell clone thereby enables the determination of their BCR specificity.

### EXAMPLE 4

Further steps of the method of the disclosure may comprise characterization of the monoclonal antibody produced by each B cell clone such as determining the specificity of the monoclonal antibody for antigen using methods known in the art. To determine the specificity of monoclonal antibodies produced according to the method of the disclosure, any one of several methods known in the art may be used. For example, antigen arrays are now available which permit the screening of up to 100 different antigens with small volumes of undiluted tissue culture supernatant fluid from B cell clones which significantly increases the efficiency of identification of antigens for which a monoclonal antibody has specificity in parallel to monoclonal antibody production number. Briefly, purified biotinylated antigens are spotted in streptavidin-coated 96-well microtiter plates by direct contact printing using 0.2 or 0.4 mm solid printing pins. Printed plates are left unwashed and plates are stored at 4 °C until ready for use. For screening, B cell clone supernatant fluid is added directly to the wells, with an optional blocking step if desired (e.g., BSA). A biotinylated human antibody, specific for an antigen in the printed array, is used as a positive control and orientation marker. B cell clone supernatants are added to the antigen arrays and incubated overnight at 4 °C. After washing with buffer (PBS + 0.1% Tween), arrays are incubated for 2 hours with an anti-human antibody (for detecting human antibody), or an anti-murine antibody (for detecting murine antibody), labeled with a fluorophore for fluorescence detection, or an enzyme (e.g., alkaline phosphatase) and colorimetric substrate for colorimetric detection. Antigen arrays can then be analyzed with high throughput microscopy, with image capture and use of imaging software to optimize and quantitate detection.

Similar high throughput antigen microarrays have been described in which more than 25,000 antigen-antibody reactivity tests can be performed in less than a week. In one system, arrays of protein antigens are covalently bound to aldehyde-coated glass slides. Binding of the antigens onto aldehyde glass slides required 24 hours to become stable, and stability was demonstrated for at least six months. The antigen microarray chips were printed on glass slides using solid pin deposition technology and a commercially available robotic system. Using this system allows as little as 20 µl of culture supernatant fluid to be incubated with antigen on the array surface. The presence of human antibodies bound to the array may be made with a mixture of anti-human IgG and anti-human IgM antibodies, conjugated to two different and distinguishable detection molecules if determination of the immunoglobulin class of the monoclonal antibody is desired simultaneously with determination of antigen specificity (e.g., laser scanning by an array reader which can incorporate three to four different lasers for differential detection).

Further steps of the method of the disclosure may comprise characterization of the monoclonal antibody such as isolating the monoclonal antibody comprising (i) purification of the monoclonal antibody from the culture medium using methods known in the art, and/or (ii) comprise isolating the total RNA from the B cell clone to produce cDNA encoding the variable-heavy (VH) antibody chains and cDNA encoding variable-light (VL) antibody chains which then can be cloned into an eukaryotic expression vector for recombinant production of the monoclonal antibody using methods known in the art. In that regard, human B cell clones expanded and differentiated using the method of the disclosure may be harvested, followed by extraction of total RNA from cells of a B cell clone. Any one of a number of methods known in the art to isolate total RNA may be used, such as using a commercially available miniprep kit. Human VH and VL chain genes may be selectively amplified using primers known in the art (see, e.g., SEQ ID NOs: 5-12) and reverse transcriptasepolymerase chain reaction to make respective cDNA molecules. Human immunoglobulin (Ig) heavy (H) and light (L) chain transcripts can be amplified using nested PCR primers. Immunoglobulinspecific cDNA is then used as a template in a nested PCR amplification. Briefly, 1-2 µl of cDNA from each monoclonal B cell expansion is used as a template with the external VH PCR primers and appropriate constant region primer (see, e.g., SEQ ID NOs: 42-67) to amplify isotype-specific BCR transcripts. If necessary, PCR products from the external amplification can be used as templates for a second round of internal amplification (see, e.g., SEQ ID NOs: 13-41). In either case, plasmidspecific sequences can be added to the forward and reverse primers to generate antibody transcripts that can be easily cloned into a vector of choice for re-expression analyses. Productive Ig rearrangements could be compared against germline Ig sequences using publicly available software, and analyzed using commercially available software to determine V(D)J gene family usage. Mutation frequencies could be determined using germline V, D, and J sequences. V_{H}-D-J_{H}, V_{K}-J_{K} and V 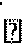 -J 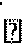 transcript alignments and phylogenetic trees based on average percent identity could be constructed using commercially available software. Once characterized, VH and VL sequences could be used to generate recombinant human or mouse monoclonal antibodies of the appropriate isotypes as needed, with subsequent expression and monoclonal antibody protein production in eukaryotic cells. For example, VH chain cDNA and VL cDNA may be cloned into expression vectors selected for the host cell to be used for expression, and the expression vectors are co-transfected into the host cell. Any number of host cells may be used to produce recombinant human or murine monoclonal antibody including, but not limited to mammalian cells (e.g., 293T cells, Chinese Hamster Ovary cells, and the like), baculovirus, insect cells, bacteria cells, and plant cells. The transfected host cells are then cloned and grown under sufficient conditions and for a sufficient time to produce antibodies. The production process can be scaled up to make large quantities of antibody, using methods known in the art and standard industrial systems. Monoclonal antibody may then be purified using standard immunopurification techniques known in the art, e.g. such as by using protein A and/or protein G chromatography (for IgG) or using anti-immunoglobulin antibody specific for IgA, IgM, or IgD as desired.

### EXAMPLE 5

In this Example, illustrated is a method for producing human monoclonal antibodies that bind to a particular antigen, typically a known antigen, wherein exposure to the antigen occurs *in vivo,* and wherein a human individual has B cells which can produce antibody having binding specificity for this antigen as the result of exposure to this antigen *in vivo.* In one illustrated application of this method, an individual may have a pathological condition, disorder, or disease process that results in exposure of that individual's B cells to one or more antigens associated with or caused by the pathological condition, disorder, or disease process. An antibody produced by such exposure may either contribute to the development or progression of the condition, disorder, or disease process ("pathological antibody") or, to the contrary, may be produced in an attempt to inhibit or prevent the development of the condition or disease ("therapeutic antibody"). The method comprises the steps of isolating B cells from such a human individual, separating the isolated B cells into single cells, culturing *in vitro* an isolated B cell (single cell) in the presence of modified feeder cells according to the disclosure under conditions and for a sufficient time to achieve at least a 10⁴-fold expansion in number in less than 2 weeks in culture, wherein produced from the expanded B cell clone in culture is monoclonal antibody. Each monoclonal antibody, produced from this co-culture, may then be assessed for antigenic specificity.

As an illustration of this method, produced were monoclonal antibodies from B cells isolated from individuals with pemphigus vulgaris (PV) or pemphigus foliaceus (PF). Individuals having either of these diseases were diagnosed by clinical presentation, histology and direct immunofluorescence findings. This study was conducted with Institutional Review Board approval, including written informed consent. Individuals enrolled in this study were pemphigus patients that were not receiving any treatment for internal or inflammatory disorders other than pemphigus. Using methods described herein, isolated B cells and the modified feeder cells were co-cultured *in vitro.* Briefly, circulating blood B cells from patients with pemphigus were isolated. MS5^{Trio} cells were cultured in 96-well plates for at least 12 hours prior to the addition of the isolated and purified blood B cells from pemphigus patients. For limiting dilution, 0.2 mL of fresh tissue culture medium containing B cells (10 cells/mL) was added to each well without any addition of exogenous cytokines. Half of the culture medium was replaced with fresh medium on days 6 and 9. Culture supernatant fluid was collected on day 12 from each well, and the supernatant fluid was assessed for the presence of autoantibody associated with pemphigus. In that regard, both forms of pemphigus (PV and PF) are caused by autoantibodies to cell surface antigens of stratified epithelia or mucous membranes and skin. Typically, these pathological antibodies bind to calcium-dependent adhesion molecules on cell surface desmosomes, notably desmoglein 1 (DSG1) in PF, and desmoglein 3 (DSG3) and/or desmogelin 1 in PV. Thus, the supernatant fluid was tested for the presence of human anti-DSG1 and human anti-DSG3 antibody by enzyme linked immunosorbent assays (ELISA). As shown in Fig. 5A, in examination of single 96-well plates, on average there were three to four wells containing a B cell clone that produced human anti-DSG1 antibody as determined by ELISA. As eleven 96-well plates were used for each patient sample, theoretically 10,560 B cells were seeded. Based on the number of positive wells from all the eleven plates and the cloning efficiency of the single B cell culture system, the proportion of antigen-specific blood B cells was calculated. As shown in Fig. 5B, about 0.3% to 0.5% of circulating B cells have antigen specificity for DSG1 or DSG3 in for pemphigus patients (Fig. 5B, PV and PF). B cell clones reactive with DSG1 and DSG3 in ELISA were also isolated from a healthy individual ("Healthy Control", HC) without pemphigus who did not have measurable anti-DSG serum antibodies (Fig. 5B Thus, demonstrated is an *in vitro* method for expanding single B cells and inducing the B cells to produce measurable human monoclonal antibody that binds to a particular antigen, typically a known antigen, wherein exposure to the antigen occurs *in vivo.* Alternatively, naïve B cells with the capacity to bind said antigen can be identified and their monoclonal antibody product isolated.

## Claims

1. A method for expanding B cells *in vitro,* the method comprising:
(a) isolating at least one B cell, and
(b) culturing the at least one B cell from step (a) with a feeder cell line comprising a thymic epithelial cell line modified to express a CD154 polypeptide (or CD40 agonist) and a BLyS polypeptide (or B cell survival factor), in combination with IL-21 and without additional exogenous IL-4, wherein the B cells are cultured with the feeder cell line under sufficient conditions and for a sufficient time to cause the human B cells to expand in number, wherein the B cells are optionally human B cells, wherein the IL-21 is optionally added exogenously to the culture or the feeder cell line is modified to express an IL-21 polypeptide, and wherein the at least one B cell has been exposed to an antigen prior to isolation in step (a), wherein one B cell is isolated in step (a) for use in step (b).

2. The method of claim 1, wherein the B cells are expanded at least an average of 10⁴-fold in number and optionally wherein the culturing of the at least one B cell with the feeder cell line in step (b) is performed in less than 2 weeks.

3. The method of any one of claims 1-2, wherein the culturing of the at least one B cell with the feeder cell line in step (b) is performed in the presence of an antigen or wherein the culturing of step (b) is performed in the absence of added antigen.

4. The method of any one of claims 1-3, wherein the at least one B cell is isolated by binding to an antigen.

5. The method of any one of claims 1-4, wherein after step (b) at least 10% of the expanded B cells are expressing IgG or wherein after step (b) at least 2.5% of the expanded B cells are expressing IgA.

6. The method of any one of claims 1-5, wherein the feeder cells are a feeder cell line comprising a thymic epithelial cell line modified to express a CD154 polypeptide, a BLyS polypeptide, and, optionally, an IL-21 polypeptide and optionally wherein the feeder cell line is capable of expanding human B cells to at least an average of 10⁴-fold in number in less than 2 weeks in culture; and optionally wherein the feeder cell line comprises the CD154 polypeptide comprises SEQ ID NO: 68 or a functional fragment or variant thereof, wherein the BLyS polypeptide comprises SEQ ID NO: 69 or a functional fragment or variant thereof, and wherein the IL-21 polypeptide comprises SEQ ID NO: 70 or a functional fragment or variant thereof.

7. An *in vitro* method for producing a monoclonal antibody comprising:
(a) isolating B cells;
(b) separating the B cells from step (a) into single B cells;
(c) culturing the single B cells with a feeder cell line comprising a stromal cell line modified to express a CD154 polypeptide (or CD40 agonist) and a BLyS polypeptide (or B cell survival factor), in combination with IL-21 and without additional exogenous IL-4 to produce a plurality of B cell clones,
wherein the single B cells are cultured with the feeder cell line under sufficient conditions and for a sufficient time to cause the single B cells to expand in number and to differentiate into a B cell clone producing a monoclonal antibody, wherein the feeder cells are a feeder cell line comprising a stromal or thymic epithelial cell line modified to express a CD154 polypeptide, a BLyS polypeptide, and, optionally, an IL-21 polypeptide and optionally wherein the feeder cell line is capable of expanding human B cells to at least an average of 10⁴-fold in number in less than 2 weeks in culture; and optionally wherein the feeder cell line comprises the CD154 polypeptide comprises SEQ ID NO: 68 or a functional fragment or variant thereof, wherein the BLyS polypeptide comprises SEQ ID NO: 69 or a functional fragment or variant thereof, and wherein the IL-21 polypeptide comprises SEQ ID NO: 70 or a functional fragment or variant thereof and optionally further comprising
(d) assessing the antigen specificity of at least one of the monoclonal antibodies produced by the plurality of B cell clones, and optionally further comprising
(e) purifying at least one of the monoclonal antibodies produced by the plurality of B cell clones.

8. The method of claim 7, wherein the B cells have been exposed to an antigen prior to isolation in step (a).

9. The method of any one of claims 7-8, wherein the B cells are human B cells or wherein the B cells are murine B cells or a subset of murine B cells.

10. The method of any one of claims 7-9, wherein antigen is not included in step (c).

11. The method of any one of claims 7-10, wherein after step (c) at least 10% of the expanded B cells are expressing IgG or wherein after step (c) at least 2.5% of the expanded B cells are expressing IgA.

12. A kit for expanding and differentiating mammalian B cells in vitro comprising feeder cells, at least one antibody for isolating B cells from a biological sample and reagents for collecting the B cells from a peripheral blood sample, wherein the feeder cells are a feeder cell line comprising a thymic epithelial or stromal cell line modified to express a CD154 polypeptide, a BLyS polypeptide, and, optionally, an IL-21 polypeptide and optionally wherein the feeder cell line is capable of expanding human B cells to at least an average of 10⁴-fold in number in less than 2 weeks in culture; and optionally wherein the feeder cell line comprises the CD154 polypeptide comprises SEQ ID NO: 68 or a functional fragment or variant thereof, wherein the BLyS polypeptide comprises SEQ ID NO: 69 or a functional fragment or variant thereof, and wherein the IL-21 polypeptide comprises SEQ ID NO: 70 or a functional fragment or variant thereof, wherein the antibody is optionally a CD19 antibody, optionally further comprising one or more of reagents necessary for culturing the modified feeder cells and the isolated B cells optionally IL-21, optionally further comprising one or more reagents for characterization of monoclonal antibodies produced from B cells, optionally an antibody specific for IgM, IgG, IgA, or IgE.

13. A feeder cell line comprising a thymic epithelial cell line modified to express a CD154 polypeptide, a BLyS polypeptide, and, optionally, an IL-21 polypeptide and optionally wherein the feeder cell line is capable of expanding human B cells to at least an average of 10⁴-fold in number in less than 2 weeks in culture.

14. The feeder cell line of claim 13, wherein the CD154 polypeptide comprises SEQ ID NO: 68 or a functional fragment or variant thereof, wherein the BLyS polypeptide comprises SEQ ID NO: 69 or a functional fragment or variant thereof, and wherein the IL-21 polypeptide comprises SEQ ID NO: 70 or a functional fragment or variant thereof.

## Patentansprüche

1. Verfahren zum Vermehren von B-Zellen in vitro, wobei das Verfahren Folgendes umfasst:
(a) das Isolieren von zumindest einer B-Zelle und
(b) das Kultivieren der zumindest einen B-Zelle aus Schritt (a) mit einer Nährzelllinie, die eine Thymusepithelzelllinie umfasst, die so modifiziert ist, dass sie ein CD154-Polypeptid (oder einen CD40-Agonisten) und ein BLyS-Polypeptid (oder einen B-Zellen-Überlebensfaktor) in Kombination mit IL-21 und ohne zusätzliches endogenes IL-4 exprimiert, wobei die B-Zellen mit der Nährzelllinie unter geeigneten Bedingungen und über einen ausreichenden Zeitraum kultiviert werden, damit sich die menschlichen B-Zellen vermehren, wobei die B-Zellen gegebenenfalls menschliche B-Zellen sind, wobei das IL-21 gegebenenfalls exogen zu der Kultur zugesetzt wird oder die Nährzelllinie so modifiziert ist, dass sie ein IL-21-Polypeptid exprimiert, und wobei die zumindest eine B-Zelle vor der Isolierung in Schritt (a) einem Antigen ausgesetzt wurde, wobei eine B-Zelle in Schritt (a) zur Verwendung in Schritt (b) isoliert wird.

2. Verfahren nach Anspruch 1, wobei sich die B-Zellen zumindest im Durchschnitt um den Faktor 10⁴ vermehren und wobei gegebenenfalls das Kultivieren der zumindest einen B-Zelle mit der Nährzelllinie in Schritt (b) innerhalb von weniger als 2 Wochen durchgeführt wird.

3. Verfahren nach einem der Ansprüche 1 bis 2, wobei das Kultivieren der zumindest einen B-Zelle mit der Nährzelllinie in Schritt (b) in Gegenwart eines Antigens durchgeführt wird oder wobei das Kultivieren von Schritt (b) in Abwesenheit eines zugesetzten Antigens durchgeführt wird.

4. Verfahren nach einem der Ansprüche 1 bis 3, wobei die zumindest eine B-Zelle durch Bindung an ein Antigen isoliert wird.

5. Verfahren nach einem der Ansprüche 1 bis 4, wobei nach Schritt (b) zumindest 10 % der vermehrten B-Zellen IgG exprimieren oder wobei nach Schritt (b) zumindest 2,5 % der vermehrten B-Zellen IgA exprimieren.

6. Verfahren nach einem der Ansprüche 1 bis 5, wobei die Nährzellen eine Nährzelllinie sind, die eine Thymusepithelzelllinie umfasst, die so modifiziert ist, dass sie ein CD154-Polypeptid, ein BLyS-Polypeptid und gegebenenfalls ein IL-21-Polypeptid exprimiert, und wobei gegebenenfalls die Nährzelllinie in der Lage ist, menschliche B-Zellen in Kultur in weniger als 2 Wochen auf das zumindest im Durchschnitt 10⁴-Fache zu vermehren; und wobei gegebenenfalls die Nährzelllinie das CD154-Polypeptid umfasst, das SEQ ID NO: 68 oder ein funktionelles Fragment oder eine Variante davon umfasst, das BLyS-Polypeptid SEQ ID NO: 69 oder ein funktionelles Fragment oder eine Variante davon umfasst und das IL-21-Polypeptid SEQ ID NO: 70 oder ein funktionelles Fragment oder eine Variante davon umfasst.

7. In-vitro-Verfahren zur Erzeugung eines monoklonalen Antikörpers, das Folgendes umfasst:
(a) das Isolieren von B-Zellen;
(b) das Trennen der B-Zellen aus Schritt (a) in einzelne B-Zellen;
(c) das Kultivieren der einzelnen B-Zellen mit einer Nährzelllinie, die eine Stromazelllinie umfasst, die so modifiziert ist, dass sie ein CD154-Polypeptid (oder einen CD40-Agonisten) und ein BLyS-Polypeptid (oder einen B-Zellen-Überlebensfaktor) in Kombination mit IL-21 und ohne zusätzliches endogenes IL-4 exprimiert, um eine Vielzahl von B-Zellklonen zu erzeugen,
wobei die einzelnen B-Zellen mit der Nährzelllinie unter geeigneten Bedingungen und über einen ausreichenden Zeitraum kultiviert werden, damit sich die einzelnen B-Zellen vermehren und zu einem B-Zellklon differenzieren, der einen monoklonalen Antikörper produziert, wobei die Nährzellen eine Nährzelllinie sind, die eine Stroma- oder Thymusepithelzelllinie umfasst, die so modifiziert ist, dass sie ein CD154-Polypeptid, ein BLyS-Polypeptid und gegebenenfalls ein IL-21-Polypeptid exprimiert, und ggf wobei gegebenenfalls die Nährzelllinie in der Lage ist, menschliche B-Zellen in Kultur in weniger als 2 Wochen auf das zumindest im Durchschnitt 10⁴-Fache zu vermehren; und wobei gegebenenfalls die Nährzelllinie das CD154-Polypeptid umfasst, das SEQ ID NO: 68 oder ein funktionelles Fragment oder eine Variante davon umfasst, das BLyS-Polypeptid SEQ ID NO: 69 oder ein funktionelles Fragment oder eine Variante davon umfasst und das IL-21-Polypeptid SEQ ID NO: 70 oder ein funktionelles Fragment oder eine Variante davon umfasst, wobei das Verfahren außerdem Folgendes umfasst:
(d) das Bewerten der Antigenspezifität von zumindest einem der von der Vielzahl von B-Zellklonen produzierten monoklonalen Antikörper, und wobei das Verfahren gegebenenfalls außerdem Folgendes umfasst:
(e) das Reinigen von zumindest einem der von der Vielzahl von B-Zellklonen produzierten monoklonalen Antikörper.

8. Verfahren nach Anspruch 7, wobei die B-Zellen vor der Isolierung in Schritt (a) einem Antigen ausgesetzt wurden.

9. Verfahren nach einem der Ansprüche 7 bis 8, wobei die B-Zellen menschliche B-Zellen sind oder wobei die B-Zellen murine B-Zellen oder eine Untergruppe von murinen B-Zellen sind.

10. Verfahren nach einem der Ansprüche 7 bis 9, wobei das Antigen in Schritt (c) nicht umfasst ist.

11. Verfahren nach einem der Ansprüche 7 bis 10, wobei nach Schritt (c) zumindest 10 % der vermehrten B-Zellen IgG exprimieren oder wobei nach Schritt (c) zumindest 2,5 % der vermehrten B-Zellen IgA exprimieren.

12. Set zum Vermehren und Differenzieren von Säugetier-B-Zellen in vitro, das Nährzellen, zumindest einen Antikörper zum Isolieren von B-Zellen aus einer biologischen Probe und Reagenzien zum Erhalt der B-Zellen aus einer Peripherblutprobe umfasst, wobei die Nährzellen eine Nährzelllinie sind, die eine Stroma- oder Thymusepithelzelllinie umfasst, die so modifiziert ist, dass sie ein CD154-Polypeptid, ein BLyS-Polypeptid und gegebenenfalls ein IL-21-Polypeptid exprimiert, und wobei gegebenenfalls die Nährzelllinie in der Lage ist, menschliche B-Zellen in Kultur in weniger als 2 Wochen auf das zumindest im Durchschnitt 10⁴-Fache zu vermehren; und wobei gegebenenfalls die Nährzelllinie das CD154-Polypeptid umfasst, das SEQ ID NO: 68 oder ein funktionelles Fragment oder eine Variante davon umfasst, das BLyS-Polypeptid SEQ ID NO: 69 oder ein funktionelles Fragment oder eine Variante davon umfasst und das IL-21-Polypeptid SEQ ID NO: 70 oder ein funktionelles Fragment oder eine Variante davon umfasst, wobei der Antikörper gegebenenfalls ein CD19-Antikörper ist, wobei gegebenenfalls das Set außerdem ein oder mehrere Reagenzien umfasst, die zur Kultivierung der modifizierten Nährzellen und der isolierten B-Zellen und gegebenenfalls IL-21 erforderlich sind, wobei das Set gegebenenfalls ein oder mehrere Reagenzien zur Charakterisierung von von B-Zellen produzierten monoklonalen Antikörpern und gegebenenfalls einen Antikörper, der für IgM, IgG, IgA oder IgE spezifisch ist, umfasst.

13. Nährzelllinie, die eine Thymusepithelzelllinie umfasst, die so modifiziert ist, dass sie ein CD154-Polypeptid, ein BLyS-Polypeptid und gegebenenfalls ein IL-21-Polypeptid exprimiert, und wobei gegebenenfalls die Nährzelllinie in der Lage ist, menschliche B-Zellen in Kultur in weniger als 2 Wochen auf das zumindest im Durchschnitt 10⁴-Fache zu vermehren.

14. Nährzelllinie nach Anspruch 13, wobei das CD154-Polypeptid SEQ ID NO: 68 oder ein funktionelles Fragment oder eine Variante davon umfasst, wobei das BLyS-Polypeptid SEQ ID NO: 69 oder ein funktionelles Fragment oder eine Variante davon umfasst und wobei das IL-21-Polypeptid SEQ ID NO: 70 oder ein funktionelles Fragment oder eine Variante davon umfasst.

## Revendications

1. Procédé d'expansion de cellules B *in vitro,* le procédé comprenant les étapes consistant à :
(a) isoler au moins une cellule B, et
(b) mettre en culture la au moins une cellules B de l'étape (a) avec une lignée de cellules nourricières comprenant une lignée de cellules épithéliales thymiques modifiée pour exprimer un polypeptide CD154 (ou un agoniste CD40) et un polypeptide BLyS (ou un facteur de survie de cellules B), en combinaison avec de l'IL-21 et sans IL-4 exogène supplémentaire, dans lequel les cellules B sont cultivées avec la lignée de cellules nourricières sous des conditions suffisantes et pendant un temps suffisant pour induire une expansion en nombre des cellules B humaines, dans lequel les cellules B sont facultativement des cellules B humaines, dans lequel de l'IL-21 est facultativement ajoutée de manière exogène à la culture ou la lignée de cellules nourricières est modifiée pour exprimer un polypeptide IL-21, et dans lequel les une ou plusieurs cellules B ont été exposées à un antigène avant un isolement lors de l'étape (a), dans lequel une cellule B est isolée lors de l'étape (a) pour être utilisée lors de l'étape (b).

2. Procédé selon la revendication 1, dans lequel les cellules B sont expansées en moyenne d'au moins 10⁴ fois en nombre et facultativement dans lequel la mise en culture de la au moins une cellule B avec la lignée de cellules nourricières dans l'étape (b) est effectuée en moins de 2 semaines.

3. Procédé selon l'une quelconque des revendications 1 à 2, dans lequel la mise en culture de la au moins une cellule B avec la lignée de cellules nourricières dans l'étape (b) est effectuée en présence d'un antigène ou dans lequel la culture de l'étape (b) est effectuée en l'absence d'antigène ajouté.

4. Procédé selon l'une quelconque des revendications 1 à 3, dans lequel au moins une cellule B est isolée par liaison à un antigène.

5. Procédé selon l'une quelconque des revendications 1 à 4, dans lequel, après l'étape (b), au moins 10 % des cellules B expansées expriment de l'IgG ou dans lequel, après l'étape (b), au moins 2,5 % des cellules B expansées expriment de l'IgA.

6. Procédé selon l'une quelconque des revendications 1 à 5, dans lequel les cellules nourricières sont une lignée de cellules nourricières comprenant une lignée de cellules épithéliales thymiques modifiée pour exprimer un polypeptide CD154, un polypeptide BLyS, et, facultativement, un polypeptide IL-21 et facultativement dans lequel la lignée de cellules nourricières est capable d'expanser des cellules B humaines à au moins une moyenne de 10⁴ fois en nombre en moins de 2 semaines en culture ; et facultativement dans lequel la lignée de cellules nourricières comprenant le polypeptide CD154 comprend SEQ ID NO : 68 ou un fragment fonctionnel ou un variant de celui-ci, dans lequel le polypeptide BLyS comprend SEQ ID NO : 69 ou un fragment fonctionnel ou un variant de celui-ci, et dans lequel le polypeptide IL-21 comprend SEQ ID NO : 70 ou un fragment fonctionnel ou un variant de celui-ci.

7. Procédé *in vitro* de production d'un anticorps monoclonal, comprenant les étapes consistant à :
(a) isoler des cellules B ;
(b) séparer les cellules B de l'étape (a) en cellules B uniques ;
(c) mettre en culture les cellules B uniques avec une lignée de cellules nourricières comprenant une lignée de cellules stromales modifiée pour exprimer un polypeptide CD154 (ou un agoniste de CD40) et un polypeptide BLyS (ou un facteur de survie de cellules B), en combinaison avec de l'IL-21 et sans IL-4 exogène supplémentaire pour produire une pluralité de clones de cellule B,
dans lequel les cellules B uniques sont mises en culture avec la lignée de cellules nourricières sous des conditions suffisantes et pendant un temps suffisant pour induire une expansion en nombre pour différentier en un clone de cellule B produisant un anticorps monoclonal, dans lequel les cellules nourricières sont une lignée de cellules nourricières comprenant une lignée de cellules épithéliales stromales ou thymiques modifiée pour exprimer un polypeptide CD154, un polypeptide BLyS, et, facultativement, un polypeptide IL-21 et facultativement dans lequel la lignée de cellules nourricières est capable d'*'*expanser des cellules B humaines à au moins une moyenne de 10⁴ fois en nombre en moins de 2 semaines en culture ; et facultativement dans lequel la lignée de cellules nourricières comprenant le polypeptide CD154 comprend SEQ ID NO : 68 ou un fragment fonctionnel ou un variant de celui-ci, dans lequel le polypeptide BLyS comprend SEQ ID NO : 69 ou un fragment fonctionnel ou un variant de celui-ci, et dans lequel le polypeptide IL-21 comprend SEQ ID NO : 70 ou un fragment fonctionnel ou un variant de celui-ci et comprenant en outre facultativement l'étape consistant à
(d) évaluer la spécificité antigénique d'au moins un des anticorps monoclonaux produits par la pluralité de clones de cellules B, et comprenant facultativement en outre l'étape consistant à
(e) purifier au moins un des anticorps monoclonaux produits par la pluralité de clones de cellule B.

8. Procédé selon la revendication 7, dans lequel les cellules B ont été exposées à un antigène avant isolement dans l'étape (a).

9. Procédé selon l'une quelconque des revendications 7 à 8, dans lequel les cellules B sont des cellules B humaines ou dans lequel les cellules B sont des cellules B murines ou un sous-ensemble de cellules B murines.

10. Procédé selon l'une quelconque des revendications 7 à 9, dans lequel l'antigène n'est pas inclus dans l'étape (c) .

11. Procédé selon l'une quelconque des revendications 7 à 10, dans lequel après l'étape (c) au moins 10 % des cellules B expansées expriment de l'IgG ou dans lequel après l'étape (c), au moins 2,5 % des cellules B expansées expriment de l'IgA.

12. Kit pour l'expansion et la différenciation de cellules B de mammifère *in vitro* comprenant des cellules nourricières, au moins un anticorps pour isoler des cellules B à partir d'un échantillon biologique et des réactifs pour collecter les cellules B à partir d'un échantillon sanguin périphérique, dans lequel les cellules nourricières sont une lignée de cellules nourricières comprenant une lignée de cellules épithéliales ou stromales thymiques modifiée pour exprimer un polypeptide CD154, un polypeptide BLyS, et, facultativement, un polypeptide IL-21 et facultativement dans lequel la lignée de cellules nourricières est capable d'expanser des cellules B humaines à au moins une moyenne de 10⁴ fois en nombre en moins de 2 semaines en culture ; et facultativement dans lequel la lignée de cellules nourricières comprenant le polypeptide CD154 comprend SEQ ID NO : 68 ou un fragment fonctionnel ou un variant de celui-ci, dans lequel le polypeptide BLyS comprend SEQ ID NO : 69 ou un fragment fonctionnel ou un variant de celui-ci, et dans lequel le polypeptide IL-21 comprend SEQ ID NO : 70 ou un fragment fonctionnel ou un variant de celui-ci, dans lequel l'anticorps est facultativement un anticorps CD19, comprenant facultativement en outre un ou plusieurs réactifs nécessaires à la culture des cellules nourricières modifiées et des cellules B isolées, facultativement de l'IL-21, comprenant facultativement en outre un ou plusieurs réactifs pour une caractérisation d'anticorps monoclonaux produits à partir de cellules B, facultativement un anticorps spécifique pour IgM, IgG, IgA ou IgE.

13. Lignée de cellules nourricières comprenant une lignée de cellules épithéliales thymiques modifiée pour exprimer un polypeptide CD154, un polypeptide BLyS et, facultativement, un polypeptide IL-21 et facultativement dans laquelle la lignée de cellules nourricières est capable d'expanser des cellules B humaines en moyenne au moins 10⁴ fois en nombre en moins de 2 semaines en culture.

14. Lignée de cellules nourricière selon la revendication 13, dans lequel le polypeptide CD154 comprend SEQ ID NO : 68 ou un fragment fonctionnel ou un variant de celui-ci, dans lequel le polypeptide BLyS comprend SEQ ID NO : 69 ou un fragment fonctionnel ou un variant de celui-ci, et dans lequel le polypeptide IL-21 comprend SEQ ID NO : 70 ou un fragment fonctionnel ou un variant de celui-ci.
